# EUROPEAN PATENT SPECIFICATION

(11) **EP 1 623 033 B1**
(45) Date of publication and mention of the grant of the patent: **16.11.2011**
(21) Application number: 04732589.9
(22) Date of filing: 13.05.2004
(51) Int. Cl.: C12N 15/82

(54) **PROCESS OF PRODUCING A PLASTID-TARGETED PROTEIN IN PLANT CELLS**
PROZESS ZUR HERSTELLUNG EINES PLASTIDGERICHTETEN PROTEINS IN PFLANZENZELLEN
PROCEDE DE PRODUCTION D'UNE PROTEINE CIBLEE DANS DES PLASTIDES DANS DES CELLULES VEGETALES

(30) Priority: 15.05.2003 DE 10321963
(43) Date of publication of application: 08.02.2006
(73) Proprietor: Icon Genetics GmbH, 80333 München (DE)
(72) Inventor: KLIMYUK, Victor, 06114 Halle (DE); BENNING, Gregor, 06108 Halle (DE); GILS, Mario, 06114 Halle (DE); GIRITCH, Anatoly, 06108 Halle (DE); GLEBA, Yuri, 06114 Halle (DE)
(74) Representative: Hartz, Nikolai
(86) International application number: PCT/EP2004/005151
(87) International publication number: WO 2004/101797

(56) References cited:
- WO-A-01/20011
- WO-A-01/94607
- US-A- 6 130 366
- US-B1- 6 429 359
- GAVEL Y ET AL: "A CONSERVED CLEAVAGE-SITE MOTIF IN CHLOROPLAST TRANSIT PEPTIDES" FEBS LETTERS, vol. 261, no. 2, 1990, pages 455-458, XP002292350 ISSN: 0014-5793 cited in the application

## Description

### FIELD OF THE INVENTION

The invention relates to a process of producing a heterologous protein of interest in genetically-modified plant cells, in particular in plastids thereof, and to proteins produced thereby.

### BACKGROUND OF THE INVENTION

Plants have become an attractive choice as a low cost, indefinitely scalable production system for recombinant proteins including for pharmaceutical and industrial application (Stoger et al., 2000, Plant Mol. Biol., 42, 583-590; Larrick & Thomas, 2001, Curr. Opin. Biotechnol., 12, 411-418). Most proteins especially in the pharmaceutical area are secretory, e.g. they are initially translated as protein precursors carrying signal peptides which target them to the endoplasmatic reticulum (ER) for further post-translational processing and compartmentalization. Said processing includes correct folding and assembly, disulfide bond formation and complex enzymatic modifications. However, post-translational protein modifications in ER of animal and plant cells can differ significantly, especially in glycosylation pattern, as plants synthesize different types of carbohydrates attached to glycosylation sites (Wilson, IB., 2002, Curr. Opin. Struct. Biol., 12, 569-577; Schillberg, Fischer & Emans, 2003, Cell Mol. Life Sci., 60, 433-445). In many cases it would be an advantage to avoid plant-specific post-translational modifications, especially glycosylation of plant-produced pharmaceutical proteins, as they may cause allergenic responses in patients. Chloroplasts have their own protein quality control system and, like the ER can provide for correct disulfide bond formation and protein folding (Dickson et al., 2000, J.Biol. Chem., 275, 11829-11835). Glycosylation that does not play any role in protein activity can be easily avoided by using two different approaches: a) targeting the protein of interest into different subcellular compartments for processing, for example in chloroplasts; b) expressing the protein of interest in plant plastids by engineering transplastomic plants. The latter approach is not suitable for expressing proteins requiring an N-terminal amino acid residue other than methionine (M). There was an attempt to address this issue by expressing the human secretory protein somatotropin in transplastomic tobacco as ubiquitin fusion in order to obtain the required N-terminus upon cleavage of ubiquitin (Staub et al., 2000, Nature Biotechnol., 18, 333-338). Ubiquitin fusion proteins are cleaved by ubiquitin protease immediately downstream of the C-terminal residue of ubiquitin, thus allowing production of recombinant proteins containing N-terminal residues of choice except proline (Baker, R.T., 1996,. Curr. Opin. Biotechno/. 7, 541-546). However, there is no ubuquitin-specific protease in chloroplasts and processing of ubiquitin-somatotropin fusion took place only during the protein extraction period giving a high level (up to 70%) of unprocessed somatotropin. There have been attemps (US 8,083,601; US 6,130,366) to target heterologous proteins Into plastids of plant cells after expression outside the plastids. This requires fusion of the protein of interest with an N-terminal transit peptide. The transit peptide has the function of facilitating translocation through the membranes of the plastid. Inside the plastid, the transit peptide is cleaved off by a plastid protease. Details of the cleavage sites of natural plastid-targeted plant proteins have been investigated (Gavel & Von Heijne 1990, FEBS Lett., 261, 455-458). These investigations concern well-adapted natural proteins of certain plants. Heterologous proteins, however, are not adapted to plant plastids by evolution. Therefore, there is the general problem to construct fusion proteins with a transit peptide and a heterologous protein of interest and a sequence around the prospective cleavage position for securing efficient and defined cleavage of the fusion protein for producing the desired protein of interest In high quality, notably with high N-terminal sequence accuracy.

Therefore, it is the problem of the invention to provide a process for producing a heterologous plastid-targeted protein of interest, whereby the desired N-terminal sequence of said protein of interest is readily obtained with high reliability.

This problem is solved by a process of producing a protein of interest in cells of a predetermined plant as defined in claim 1. Also described is a process of producing a protein of interest in cells of a predetermined plant, comprising: introducing into said cells a vector encoding a fusion protein comprising in the direction from the N-terminus to the C-terminus
(i) a transit peptide for targeting said fusion protein into plastids and, contiguous thereto,
(ii) said protein of interest,
   wherein the c-terminal three amino acids X⁻³X⁻²X⁻¹ of said transit peptide and the N-terminal amino acid Z of said protein of interest form a cleavage site

   X⁻³X⁻²X⁻¹-Z

   for cleaving said fusion protein between X⁻¹ and Z for releasing said protein of interest in plastids, whereby for a predetermined amino acid Z of said protein of interest an amino acid sequence X⁻³X⁻²X⁻¹ is selected from the set of amino acid sequences X⁻³X⁻²X⁻¹ naturally occurring contiguous to Z on the N-terminal side of Z in plastid-targeted fusion proteins in plants, thereby forming said cleavage site.
   Herein, the single letter code is generally used for the 20 standard amino acids. Similarly, the symbols X⁻³, X⁻², X⁻¹, and Z each stands for a standard amino acid. Numerals -1, -2, and -3 indicate the position of the respective amino acid in the sequence of the transit peptide in direction towards the N-terminus of the transit peptide.

It is generally accepted that the cleavage accuracy and efficiency is dependent on the sequence of the transit peptide and the plastid-targeted natural protein in the vicinity of the cleavage position. However, a general knowledge extendable to combinations of an arbitrary transit peptide and a selected protein of interest is not available for application to heterologous proteins.

The length of the C-terminal sequence of the transit peptide and the length of the N-terminal sequence of the protein of interest that jointly determine the cleavage position have been uncertain. For natural proteins it is reasonable to assume that these two sequences have been mutually adjusted during revolution. For a heterologous protein to be targeted to plastids, the appropriate transit peptide has to be determined by trial and error. The success of said trial and error is not predictable. With shear luck a suitable combination of sequences may be found. However, the uncertainty is great and not calculateable.

We have surprisingly found that the problem of finding a C-terminal sequence of the transit peptide adapted to the N-terminal sequence of the protein of interest can be solved with a greater success rate based on a hierarchy of considerations. Within this hierarchy, it has been found that it is sufficient to primarily consider only the N-terminal amino acid of the heterologous protein of interest and the three last amino acids of the transit peptide. Notably, it has been found that each N-terminal amino acid of the protein of interest is correlated with a certain set of suitable C-terminal amino acid triads of the transit peptide.

In the process of the invention, a vector encoding said fusion protein is introduced into plant cells. Said protein of interest may then be expressed in said plant cells e.g. in cell culture. Preferably, said protein of interst is expressed in whole plants. This may be achieved by regenerating plants form plant cells transformed with said vector. Alternatively, said vector is introduced in plant cells of a whole plant. Several transformation or transfection methods for plants or plant cells are known in the art and include *Agrobacterium*-mediated transformation, particle bombardment, PEG-mediated protoplast transformation, viral infection etc. For the preferred embodiment of transient expression or transfection for transient expression, viral infection or *Agrobacterium*-mediated transformation are advantageously employed. Plants or plant cells are transformed or transfected with a nucleotide sequence (vector) having a coding region encoding said fusion protein. Transformation may produce stably transformed plants or plant cells, e.g. transgenic plants. Alternatively, said plant or plant cells may be transfected for transient expression of said fusion protein. Transient transfection of grown up plants is most preferred.

Said vector may be a DNA or an RNA vector depending on the transformation or transfection method. In most cases, it will be DNA. In an important embodiment, however, transformation or transfection is performed using RNA virus-based vectors, in which case said nucleotide sequence may be RNA. One very convenient way is to use a DNA vector that is based on a virus. Preferably, the DNA vector is based on an RNA virus, i.e. the DNA vector contains the cDNA of RNA viral sequences in addition to said nucleotide sequence. Examples of plant DNA or RNA viruses sequences of which may be used for viral vectors according to the invention are given in WO 02/29068 and in WO0288369. Such DNA vectors further contain a transcriptional promoter for producing the RNA viral transcript. In these embodiments, transformation or transfection is preferably carried out by viral transfection, more preferably via *Agrobacterium-*mediated transformation.

For an N-terminal amino acid Z of a protein of interest to be produced by the process of the invention, an amino acid triade X⁻³X⁻²X⁻¹ is selected from the set of amino acid sequences X⁻³X^{- 2}X⁻¹ naturally occurring on the N-terminal side of Z contiguous to Z in plastid-targeted fusion proteins in plants. Preferably, said protein of interest is produced in angiosperms. In this case, X⁻³X⁻²X⁻¹ triades naturally occurring in angiosperms are selected for a predetermined Z. More preferably, the amino acid sequence (or triade) X⁻³X⁻²X⁻¹ is selected from the set of amino acid sequences X⁻³X⁻²X⁻¹ naturally occurring contiguous to Z in plastid-targeted fusion proteins in the family, more preferably the genus, of plants said predetermined plant is a member of. Most preferably, the amino acid sequence X⁻³X⁻²X⁻¹ is selected from the set of amino acid sequences X⁻³X⁻²X⁻¹ naturally occurring contiguous to Z in plastid-targeted fusion proteins of plants of the same species as said predetermined plant.

If Z is M, said triade X⁻³X⁻²X⁻¹ is preferably FRV, NRE, VQC, VRC, or VPE.
If Z is C, said triade X⁻³X⁻²X⁻¹ is RGA, SIR, TIV or VRA.
If Z is I, said triade X⁻³X⁻²X⁻¹ is AHS, GST, or VHC.
If Z is N, said triade X⁻³X⁻²X⁻¹ is GST, KAT, or KQS.
If Z is H, said triade X⁻³X⁻²X⁻¹ is GSD.
if Z is Y, said triade X⁻³X⁻²X⁻¹ is VAA.
If Z is F, said triade X⁻³X⁻²X⁻¹ is PSR or RFN.
If Z is P, said triade X⁻³X⁻²X⁻¹ is IAE, RVA, RSA,or SVD.
If Z is Q, said triade X⁻³X⁻²X⁻¹ is DDN, IRA, SLG, or PGL
If Z is G, said triade X⁻³X⁻²X⁻¹ is DSC, IIC, IVC, LRQ, SAT, VHC, VHA, or VKC.
If Z is K, said triade X⁻³X⁻²X⁻¹ is IVA, LLV, LPL, LAS, LRQ, MAA, NNN, RTD, TAE, TAQ, TEA, TSE, VAA, VEA, or VVC.
If Z is R, said triade X⁻³X⁻²X⁻¹ is AAA, IPA, MPT, or VPS.
If Z is E, said triade X⁻³X⁻²X⁻¹ is ALA, CRA, IVC, TPS, or VRA.
If Z is L, said triade X⁻³X⁻²X⁻¹ is VLA or LSR.
If Z is S, said triade X⁻³X⁻²X⁻¹ is AGA, CLS, GKR, FPI, IAG, ITC, IVA, KAM, LCM, NMT, PAK, RLR, SVS, TTR, VCM, VVA, VAQ, VCC, VRC, VCA, VRA, or SLA.
If Z is V, said triade X⁻³X⁻²X⁻¹ is KMS, PRA, PKA, SLF, STS, TGV, TRM, VSF, VRA
If Z is D, said triade X⁻³X⁻²X⁻¹ is ASA.
If Z is T, said triade X⁻³X⁻²X⁻¹ is AVA, PAA, VAA, VAG, VSA, VNN, or WPR.

Using the combinations of triades X⁻³X⁻²X⁻¹ with a predetermined N-terminal residue Z of a protein of interest as defined in claim 1, the probability of finding a suitable transit peptide with a chosen protein of interest such that the resulting fusion protein is efficiently targeted to plastids and cleaved to release the protein of interest with high accuracy of the N-terminal end is significantly increased compared to a prior art process that relies on luck. If more than one X⁻³X⁻²X⁻¹ is possible for a certain Z, the efficiencies of these X⁻³X⁻²X⁻¹ triades may differ. A particularly suitable X⁻³X⁻²X⁻¹ amino acid triade for a certain protein of interest may be seleced by an assay comprising the following steps:
(a) constructing a vector encoding a fusion protein comprising, in the direction from its N-terminus to its C-terminus, a transit peptide having the C-terminal amino acids X⁻³X⁻²X⁻¹ as defined in table 1 for said predetermined Z and, contiguous thereto, a reporter protein like green fluorescent protein having the predetermined N-terminal amino acid Z,
(b) introducing said vector in plant cells for expressing said fusion protein,
(c) assessing cleavage of said fusion protein between X⁻¹ and Z in plastids,
(d) repeating steps (a) to (c) with one or more other X⁻³X⁻²X⁻¹ as defined in claim 5 or 6 for said predetermined Z,
(e) selecting an X⁻³X⁻²X⁻¹ leading to favourable cleavage.

Instead of putting said reporter protein on the C-terminal side of X⁻³X⁻²X⁻¹ said protein of interest may be placed contiguous to X⁻³X⁻²X⁻¹ whereby said protein of interest may be followed by a reporter protein. If said protein of interest is used in said assay, the X⁻³X⁻²X⁻¹ triade selected in the assay as a higher probability of giving efficient targeting and cleaving in the process of the invention.

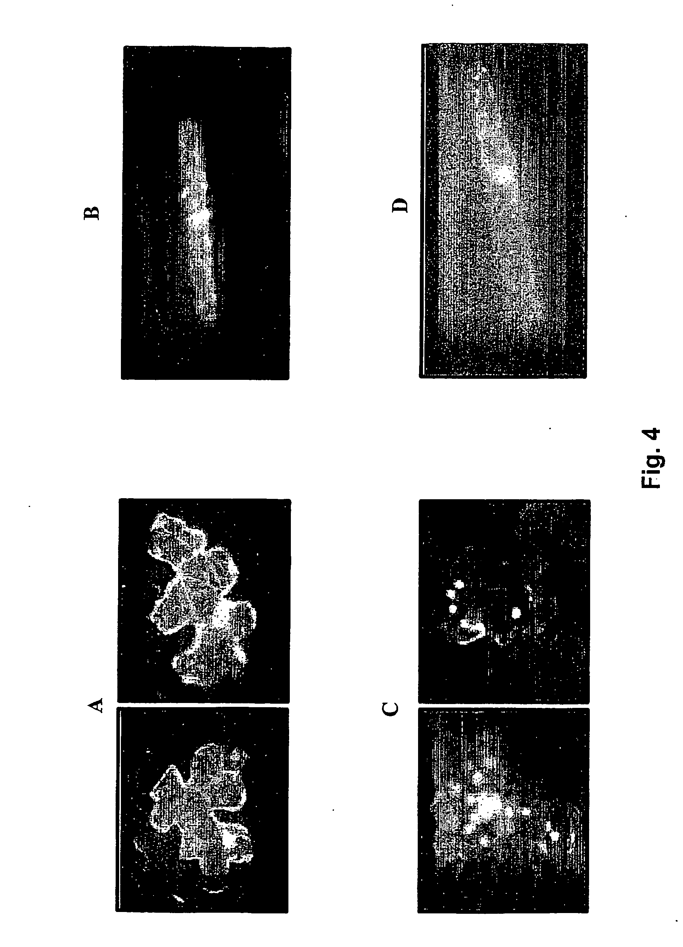

For simplicity, the assay is preferably performed in plant cell culture, whereby the cells may derive from the same plant as is used for producing the protein of interest. Alternatively, leaves of a plant may be transiently transfected with the vector of the assay. Both methods allow a straightforward assessing of targeting the fusion protein to plastids and of cleaving the fusion protein. Targeting of the fusion protein to plastids may e.g. be assessed using a fluorescent reporter protein like green fluorescent protein (GFP) and together with fluorescence microscopy as has been done for Fig. 4. Correct cleavage of the fusion protein may be checked by isolating the reporter protein or said protein of interest (that may be fused to a reporter protein like GFP) followed N-terminal sequencing. In summary, said assay allows to further increase the success rate of finding a transit peptide suitable for a protein of interest to be targeted in plastids. Regarding said transit peptide to be used in the process of the invention, there are no particular limitations. It is however of advantage to use a plastid transit peptide known from a plant that is related to the predetermined plant used for the process of the invention. If the process of the invention is carried out in dicot plant cells or in dicot plants, a highly preferred transit peptide has the sequence, in N-terminal to C-terminal direction, MASSMLSSAA WATRASAAQ ASMVAPFTGL KSAASFPVTR KQNNLDITSI ASNGGRX⁻³X⁻²X⁻¹. If the process of the invention is carried out in monocot plant cells or in monocot plants, a highly preferred transit peptide has the sequence, in N-terminal to C-terminal direction, MAPTVMASSA TTVAPFQGLK STAGRLPVAR RSSGSLGSVS NGGRX⁻³X⁻²X⁻¹.

No particular limitations exist regarding to protein of interest to be produced according to the invention. The protein of interest may be of bacterial, viral, plant, or animal origin or it may be artificially designed. Said protein of interest may be an agricultural trait, a human or animal health protein, an immune response protein, a polypeptide hormone, etc.

### BRIEF DESCRIPTION OF THE FIGURES

Figure 1. Peptide sequences of transit peptides of the small subunit of rubisco from nine different dicotyledonous plants and, at the bottom, the consensus sequence derived therefrom.
Figure 2. Peptide sequences of transit peptides of the small subunit of rubisco from six different monocotyledonous species and, at the bottom, a consensus sequence derived therefrom.
Figure 3 (A, B) depicts schematic representations of vectors pICH5300 (A) and pICH5320 (B).
Figure 4 shows transient expression of GFP in tobacco (A, C) and wheat (B, D) epidermal cells. A, B: GFP without transit peptide; C: GFP fusion with synthetic transit peptide for dicotyledonous species; D: GFP fusion with synthetic transit peptide for monocotyledonous species.
Figure 5 depicts constructs for screening for optimal X⁻³X⁻²X⁻¹ triads for a protein of interest with a given N-terminal amino acid Z. TP: sequence coding for transit peptide without the C-terminal amino acids X⁻³X⁻²X⁻¹; GOI: gene of interest; GFP: coding sequence of green fluorescent protein; pr1 and pr 2: overlapping primers for designing and cloning the region encoding X⁻³X^{- 2}X⁻¹-Z sequence: RS1 and RS2: custom restriction sites.
Figure 6 depicts the amino acid sequences of all predicted types of fusion proteins for targeting a protein of interest (somatotropin or interferon alpha-2b) into plastids by way of a synthetic transit peptide. The fusion proteins are designed to produce required N-terminal amino acid sequence of the proteins of interest after cleaving off the transit peptide. Underlined - synthetic transit peptide; boxed: predicted variants of X⁻³X⁻²X⁻¹ triads as C-terminal amino acids of the transit peptide; in bold: first amino acid sequence of protein of interest.

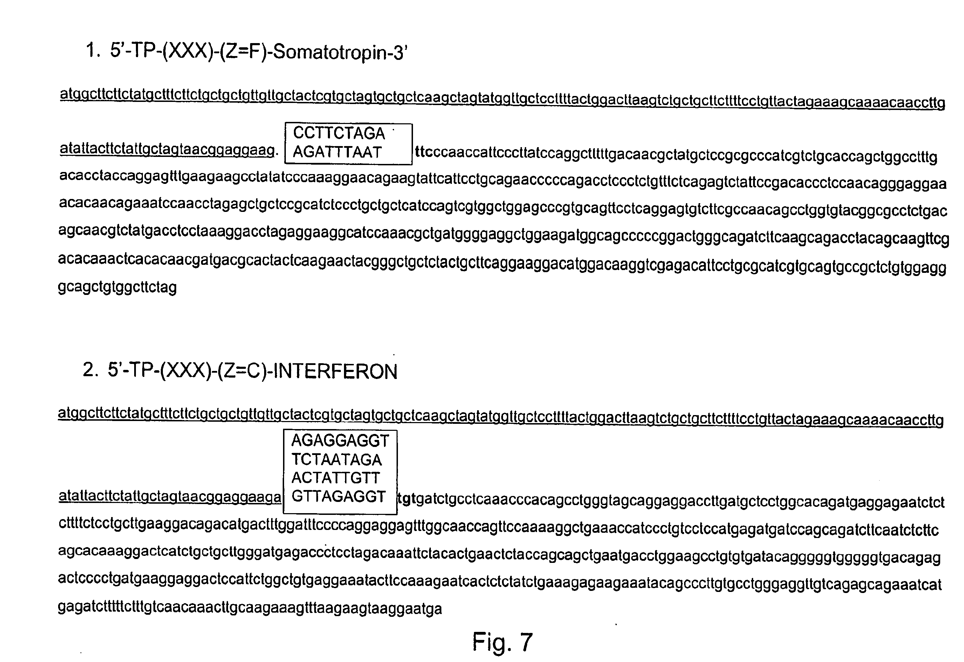

Figure 7 depicts the DNA sequences encoding for the protein fusions shown in Fig. 6.
Figure 8 depicts the schematic presentation of T-DNA regions of the binary vectors pICH14061 and pICH14071.
Figure 9 depicts the results of Western blot analysis of plastid-targeted human growth hormone (hGH) somatotropin using two different predicted variants of X⁻³X⁻²X⁻¹ triads as C-terminal amino acids of the transit peptide.
   A: Western blot with anti-hGH antibodies. Lane C - mature hGH (control); lanes 1, 2 - hGH expressed from pICH14061A; lanes 3, 4 - hGH expressed from pICH14061B. U - hGH precursor (unprocessed); M - mature, correctly processed hGH; S - incorrectly processed (small) hGH.
   B: Detailed schemes of the T-DNA regions of the binary vectors pICH14061A and pICH14061B. TP-transit peptide; P-transcriptional promoter; T-transcriptional terminator; NPT-neomycin phosphotransferase; NTR-3' non-transtated region of tobacco mosaic virus. Amino acid triads R-F-N (pICH14061A) and P-S-R (pICH14061B) are given in the one-letter amino acid code in the direction from the N-terminal side to the C-terminal side.

### DETAILED DESCRIPTION OF THE INVENTION

The general principle of the invention is the following: a gene encoding the fusion protein TP(XXX)-(Z)P (from the N-terminus to the C-terminus) comprising
(i) a chloroplast transit peptide TP(X⁻³X⁻²X⁻¹) with the C-terminal amino acid residues X⁻³X⁻²X⁻¹ and, contiguous thereto,
(ii) a protein of interest (Z)P, wherein (Z) designates the N-terminal amino acid of the protein of interest P
   is delivered into plant cells preferably using a DNA or an RNA vector. The transit peptide TP is engineered in such a way that said three C-terminal amino acid residues X⁻³X⁻²X⁻¹ of the transit peptide together with N-terminal amino acid Z of the protein of interest form a cleavage site recognized by stromal processing peptidase (Robinson & Ellis, 1984, Eur. J. Biochem., 142, 337-342). Said protein of interest is provided with the required N-terminus Z as the result of such cleavage. In order to achieve this goal, the transit peptide shall be engineered such that the choice of the amino acid residues X⁻³X⁻²X⁻¹ depends on the N-terminus Z of said protein of interest (see Table 1). Predominantly, the sets of X⁻³X⁻²X⁻¹ are unique for each N-terminal amino acid Z, except for some X⁻³X⁻²X⁻¹, which show limited degeneracy, e.g. can match different Z (shown in Table 2).

The data shown in Table 1 are the result of transit peptide cleavage site analysis for approximately 400 nuclear-encoded chloroplast targeted proteins from publicly available databases. Some X⁻³X⁻²X⁻¹ triads correspond to the cleavage motif (I/V)-X-(A/C)-A suggested by Gavel & Von Heijne 1990, FEBS Lett., 261, 455-458. For compiling Table 1, we have taken into account the possibility of 1-2 amino acid residues removal from the N-terminus after cleaving off the transit peptide (Emanuelsson, Nielsen & Heijne, Protein Sci., 8, 978-984). We found appropriate X⁻³X⁻²X⁻¹ triads for almost all possible N-terminal amino acid residues Z, except for tryptophan (trp, W). However, according to the N-end rule, W at the N-terminus destabilizes proteins in eucaryotic and prokaryotic cells, reducing the protein half-life to 2-3 min (Varshavsky, A., 1996, Proc. Natl. Acad. Sci. USA, 93, 12142-12149).

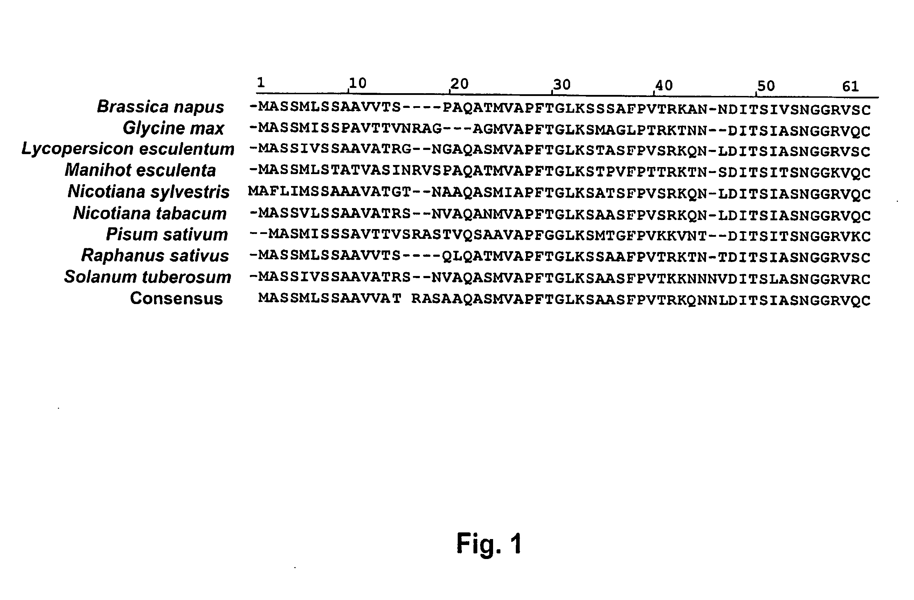

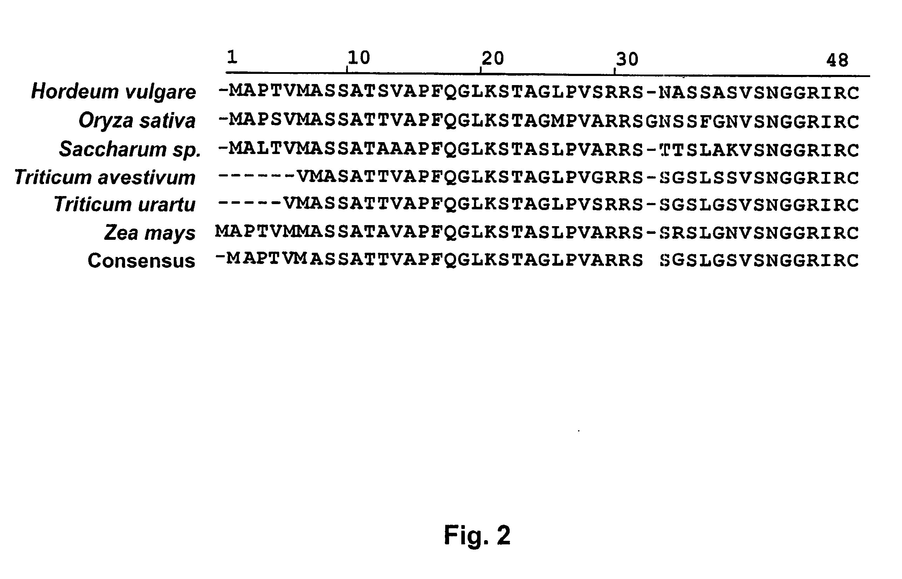

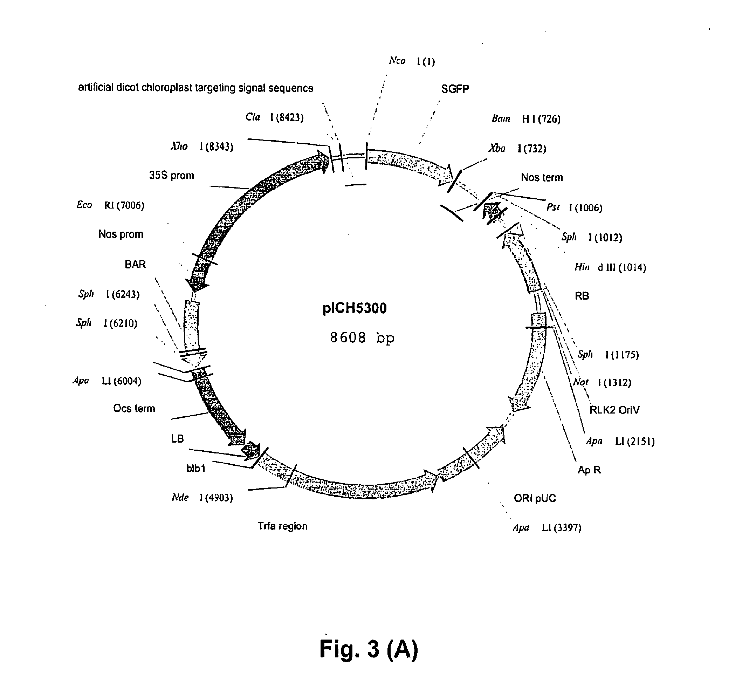

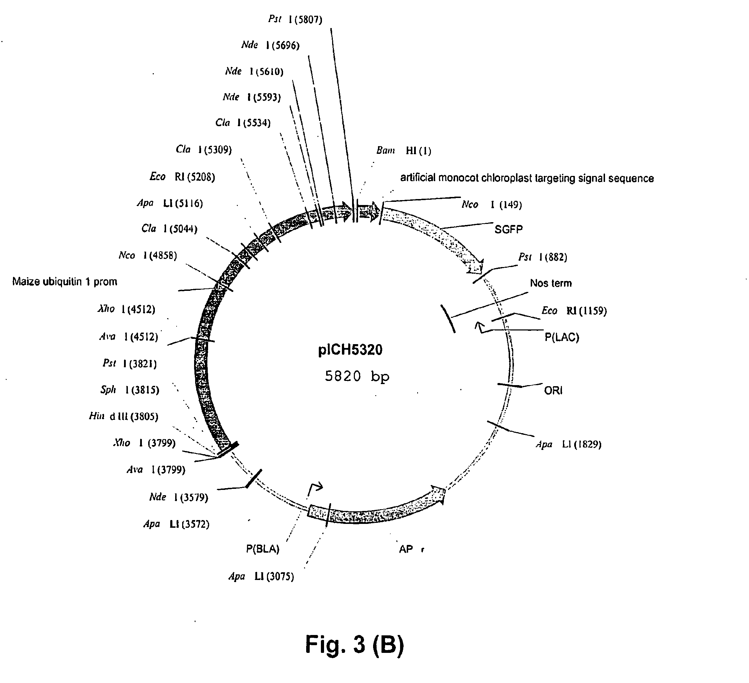

We also used sequence alignments of transit peptides of the small subunit of RUBISCO from different plant species in order to build artificial consensus transit peptides suitable for efficient targeting of proteins of interest into the plastids of dicotyledonous (Fig. 1) and monocotyledonous (Fig. 2) plants. The details of engineering such sequences and testing for their functionality are described in example 1. Constructs carrying GFP fusions with artificial transit peptides were designed (Fig. 3A,B) and tested by using microprojectile bombardment of tobacco and wheat leaves. The results shown in Figure 4 demonstrate that the artificial transit peptides given in Fig. 3A,B efficiently target the reporter protein into chloroplasts of monocotyledonous and dicotyledonous plant cells. The transit peptides were designed in such a way that they reveal minimum homology to the DNA sequences encoding the transit peptides used for building consensus, but without jeopardizing their targeting efficiency. This was done as preventive measure for avoiding possible transgene silencing caused by homology of the host-encoded transit peptide to the one of transgene.

The sequences designed as described in example 1 can be used for testing all possible combinations of a specific Z with X⁻³X⁻²X⁻¹ triads. The scheme of experiment is described in example 2.

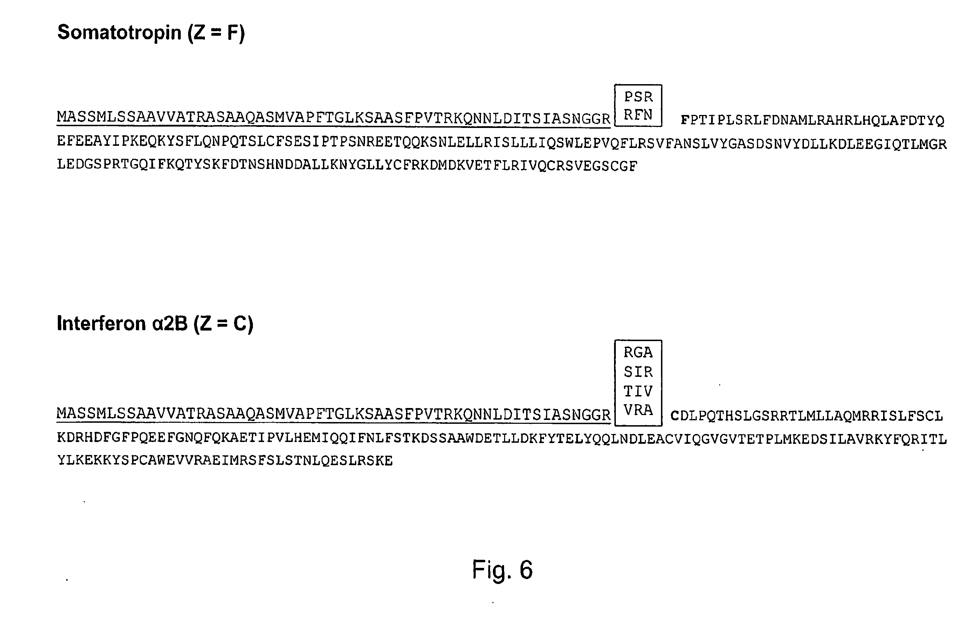

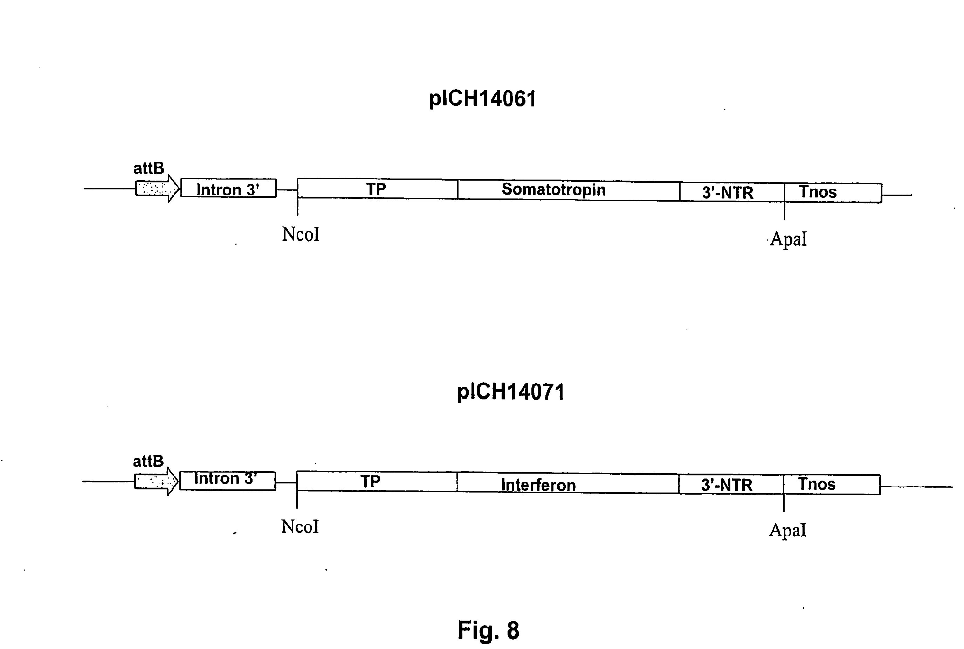

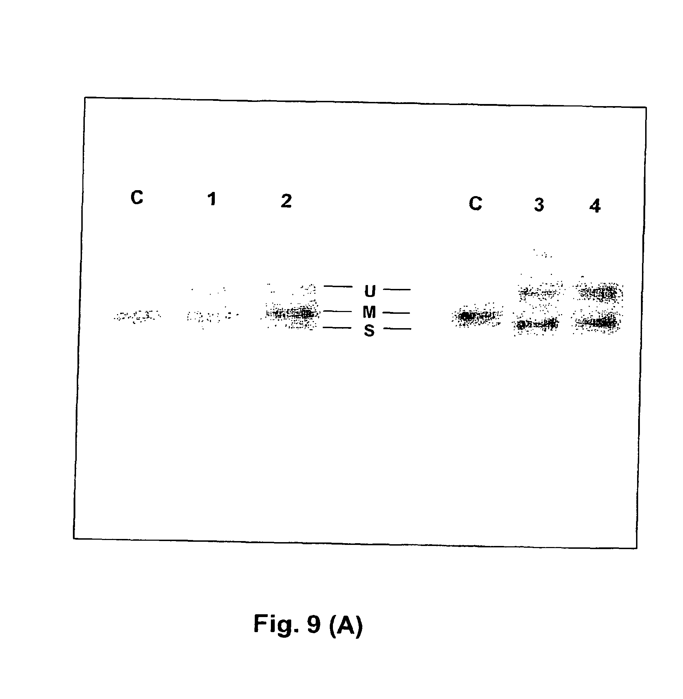

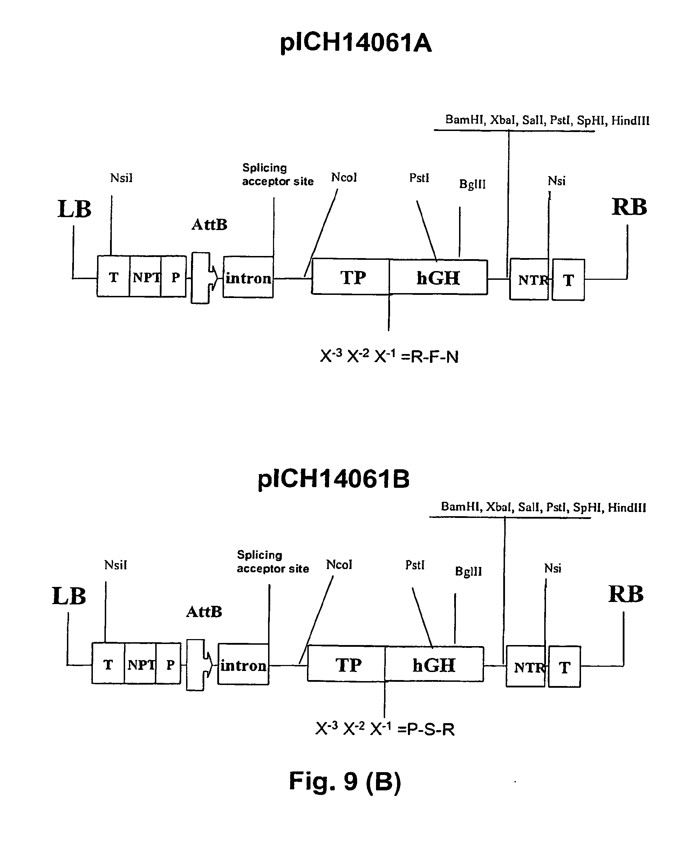

In example 3 of the invention we describe the use of the artificial transit peptide for delivery of the human growth hormone (hGH) somatotropin and human interferon alpha 2b into the chloroplasts of *Nicotiana benthamiana* plants. Both proteins are secretory and have in the processed form (after cleaving off the transit peptide) an N-terminus starting from phenylalanine (F) for somatotropin and from cysteine (C) for interferon alpha 2b. Figure 6 shows in boxes the possible X⁻³X⁻²X⁻¹ triads for the respective N-termini of the proteins of interest.The constructs coding for the fusion proteins of Fig. 6 were subcloned into the 3'provector (Fig. 8) of a viral expression system (Marillonnet et al., 2004, Proc. Natl. Acad. Sci. USA, 101, 6852-6857) and transiently expressed in *Nicotiana benthamiana* plants. Alternatively, vectors coding for said fusion proteins can be stably transformed into the plant nuclear DNA. The results of Western blot analysis shown in Fig. 9 demonstrate that one of the two triads (X⁻³X⁻²X⁻¹=R-F-N; pICH14061A) produces a major part of hGH of a size expected for the correctly processed protein, while the second fusion (X⁻³X⁻²X⁻¹=P-S-R; pICH14061B) does not show a band at the expected size, but unprocessed hGH precursor (U) and a band (S) that corresponds to an incorrectly cleaved protein that is smaller than the correctly processed mature protein. This demonstrates the validity of the approach of this invention, taking into account that both triads are not part of a predicted transit peptide cleavage site (I/V)-X-(A/C)-A suggested by Gavel & Von Heijne 1990, FEBS Lett., 261, 455-458.

Various methods can be used to deliver a DNA or RNA vector into the plant cell, including direct introduction of said vector into a plant cell by means of microprojectile bombardment, electroporation or PEG-mediated treatment of protoplasts (for review see: Gelvin, S.B., 1998, Curr. Opin. Biotechnol., 9, 227-232; Hansen & Wright, 1999, Trends Plant Sci., 4, 226-231). Plant RNA and DNA viruses are also efficient delivery systems (Hayes et al.,1988, Nature, 334, 179-182; Palmer et al., 1999, Arch. Virol., 144, 1345-1360; Lindbo et al., 2001, Curr. Opin. Plant. Biol., 4, 181-185). Said vectors can deliver a transgene either for stable integration into the genome of the plant (direct or *Agrobacterium-*mediated DNA integration) or for transient expression of the transgene ("agroinfiltration").

Preferred plants for the use in this invention include any plant species with preference given to agronomically and horticulturally important species. Common crop plants for the use in the invention include alfalfa, barley, beans, canola, cowpeas, cotton, com, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. Plant species preferred for practicing this invention include but are not restricted torepresentatives of Graminae, Compositae, Solanacea and Rosaceae.

Additionally, preferred species for use in the invention are plants from the genera: Arabidopsis, Agrostis, Allium, Antirrhinum, Apium, Arachis, Asparagus, Atropa, Avena, Bambusa, Brassica, Bromus, Browaalia, Camellia, Cannabis, Capsicum, Cicer, Chenopodium, Chichorium, Citrus, Coffea, Coix, Cucumis, Curcubita, Cynodon, Dactylis, Datura, Daucus, Digitalis, Dioscorea, Elaeis, Eleusine, Festuca, Fragaria, Geranium, Glycine, Helianthus, Heterocallis, Hevea, Hordeum, Hyoscyamus, Ipomoea, Lactuca, Lens, Lilium, Linum, Lolium, Lotus, Lycopersicon, Majorana, Malus, Mangifera, Manihot, Medicago, Nemesia, Nicotiana, Onobrychis, Oryza, Panicum, Pelargonium, Pennisetum, Petunia, Pisum, Phaseolus, Phleum, Poa, Prunus, Ranunculus, Raphanus, Ribes, Ricinus, Rubus, Saccharum, Salpiglossis, Secale, Senecio, Setaria, Sinapis, Solanum, Sorghum, Stenotaphrum, Theobroma, Trifolium, Trigonella, Triticum, Vicia, Vigna, Vitis, Zea, and the Olyreae, the Pharoideae and many others.

Within the scope of this invention, plant species which are not included into the food or feed chain are particularly preferred for producing pharmaceutical and technical proteins. Among those, Nicotiana species are the most preferred, as they are easy to transform and to cultivate with well developed expression vector (especially viral vectors) systems.

Genes of interest, their fragments (functional or non-functional) and their artificial derivatives that can be expressed as the cellular process of interest and isolated using the present invention include, but are not limited to: starch modifying enzymes (starch synthase, starch phosphorylation enzyme, debranching enzyme, starch branching enzyme, starch branching enzyme II, granule bound starch synthase), sucrose phosphate synthase, sucrose phosphorylase, polygalacturonase, polyfructan sucrase, ADP glucose pyrophosphorylase, cyclodextrin glycosyltransferase, fructosyl transferase, glycogen synthase, pectin esterase, aprotinin, avidin, bacterial levansucrase, *E.coli* glgA protein, MAPK4 and orthologues, nitrogen assimilation/methabolism enzyme, glutamine synthase, plant osmotin, 2S albumin, thaumatin, site-specific recombinase/integrase (FLP, Cre, R recombinase, Int, SSVI Integrase R, Integrase phiC31, or an active fragment or variant thereof), isopentenyl transferase, Sca M5 (soybean calmodulin), coleopteran type toxin or an insecticidally active fragment, ubiquitin conjugating enzyme (E2) fusion proteins, enzymes that metabolise lipids, amino acids, sugars, nucleic acids and polysaccharides, superoxide dismutase, inactive proenzyme form of a protease, plant protein toxins, traits altering fiber in fiber producing plants, Coleopteran active toxin from *Bacillus thuringiensis* (Bt2 toxin, insecticidal crystal protein (ICP), CryIC toxin, delta eridotoxin, polyopeptide toxin, protoxin etc.), insect specific toxin AaIT, cellulose degrading enzymes, E1 cellulase from *Acidothermus celluloticus,* lignin modifying enzymes, cinnamoyl alcohol dehydrogenase, trehalose-6-phosphate synthase, enzymes of cytokinin metabolic pathway, HMG-CoA reductase, *E*. *coli* inorganic pyrophosphatase, seed storage protein, *Erwinia herbicola* lycopen synthase, ACC oxidase, pTOM36 encoded protein, phytase, ketohydrolase, acetoacetyl CoA reductase, PHB (polyhydroxybutanoate) synthase, acyl carrier protein, napin, EA9, non-higher plant phytoene synthase, pTOM5 encoded protein, ETR (ethylene receptor), plastidic pyruvate phosphate dikinase, nematode-inducible transmembrane pore protein, trait enhancing photosynthetic or plastid function of the plant cell, stilbene synthase, an enzyme capable of hydroxylating phenols, catechol dioxygenase, catechol 2,3-dioxygenase, chloromuconate cycloisomerase, anthranilate synthase, *Brassica* AGL15 protein, fructose 1,6-biphosphatase (FBPase), AMV RNA3, PVY replicase, PLRV replicase, potyvirus coat protein, CMV coat protein, TMV coat protein, luteovirus replicase, MDMV messenger RNA, mutant geminiviral replicase, *Umbellularia californica* C12:0 preferring acyl-ACP thioesterase, plant C10 or C12:0 preferring acyl-ACP thioesterase, C14:0 preferring acyl-ACP thioesterase (IuxD), plant synthase factor A, plant synthase factor B, Δ6-desaturase, protein having an enzymatic activity in the peroxysomal β-oxidation of fatty acids in plant cells, acyl-CoA oxidase, 3-ketoacyl-CoA thiolase, lipase, maize acetyl-CoA-carboxylase, 5-enolpyruvylshikimate-3-phosphate synthase (EPSP), phosphinothricin acetyl transferase (BAR, PAT), CP4 protein, ACC deaminase, protein having posttranslational cleavage site, DHPS gene conferring sulfonamide resistance, bacterial nitrilase, 2,4-D monooxygenase, acetolactate synthase or acetohydroxyacid synthase (ALS, AHAS), polygalacturonase, Taq polymerase, bacterial nitrilase, many other enzymes of bacterial or phage including restriction endonucleases, methylases, DNA and RNA ligases, DNA and RNA polymerases, reverse trascryptases, nucleases (DNases and RNAses), phosphatases, transferases etc.

Our invention also can be used for the purpose of molecular farming and purification of commercially valuable and pharmaceutically important proteins including industrial enzymes (cellulases, lipases, proteases, phytases etc.) and fibrous proteins (collagen, spider silk protein, etc.). Any human or animal health protein can be expressed and purified using described in our invention approach. Examples of such proteins of interest include inter alia immune response proteins (monoclonal antibodies, single chain antibodies, T cell receptors etc.), antigens including those derived from pathogenic microorganisms, colony stimulating factors, relaxins, polypeptide hormones including somatotropin (HGH) and proinsulin, cytokines and their receptors, interferons, growth factors and coagulation factors, enzymatically active lysosomal enzyme, fibrinolytic polypeptides, blood clotting factors, trypsinogen, a1-antitrypsin (AAT), human serum albumin, glucocerebrosidases, native cholera toxin B as well as function-conservative proteins like fusions, mutant versions and synthetic derivatives of the above proteins.

### EXAMPLE 1

### Generation of synthetic transit peptide sequences for monocotyledonous and dicotyledonous plants

### a) transit peptide for plastid targeting in dicotyledonous plants

The consensus amino acid sequence of nine chloroplast targeting transit peptides from rubisco small subunit precursor proteins (rbcs) of different dicotyledonous plants was generated by sequence analysis with the DNASTAR software package (Figure 1). The nucleotide sequence encoding the consensus transit peptide was designed taking into account the codon usage for dicotyledonous plants: each triplet codon was selected on the basis of highest codon usage values giving an average GC-content of 43.6 %. Also, by designing the sequence we tried to maximize the difference on cDNA level between the cDNA for consensus sequence and cDNAs encoding for transit peptides of dicot species used for building the consensus. The final nucleotide sequence flanked by convenient restriction sites was *de novo* synthesized and subcloned as ClaI/NcoI-fragment (**ClaI 5'-cATCGATaac** atggcttctt ctatgctttc ttctgctgct gttgttgcta ctcgtgctag tgctgctcaa gctagtatgg ttgctccttt tactggactt aagtctgctg cttcttttcc tgttactaga aagcaaaaca accttgatat tacttctatt gctagtaacg gaggaagagt tcaatgc**gCC ATGG-3' NcoI**) into the constructs of interest in order to make N-terminal translational fusion with the reporter gene (GFP) (see Fig. 3A, plasmid pICH5300).

### b) transit peptide for targeting in monocotyledonous plants

The same strategy as described above was used to create an artificial chloroplast targeting signal sequence for the expression and plastid targeting in monocot plants. The consensus amino acid sequence derived from six chloroplast transit peptides from rbcs-proteins of different monocot plants was generated by sequence analysis with the DNASTAR software package (Figure 2). The nucleotide sequence encoding the consensus transit peptide was designed taking into account the codon usage for monocotyledonous plants: each triplet codon was selected on the basis of the highest codon usage values giving an average GC-content of 71.0 % in the final nucleotide sequence. The nucleotide sequence encoding for consensus transit peptide of monocotyledonouys species was de novo synthesized and subcloned as Cla1/Nc01 or BamHI/NcoI-fragment (**ClaI/BamHI 5'-cATCGATAGG ATCCacg**atg gccccaaccg tgatggcctc ctccgccacc accgtggccc cattccaggg cctcaagtcc accgccggcc tcccagtggc caggaggtcc tccggcagcc tcggcagcgt gagcaacggc ggcaggatca ggtgc**gCCAT GG-3'NcoI**) into the vector of interest (see Fig. 3B, plasmid pICH5320).

In order to test the efficiency of chloroplast targeting with the help of artificial transit peptides, the plasmids encoding the transit peptide - reporter gene fusion were delivered into leaf cells of tobacco and wheat with the help of microprojectile bombardment. The results showed an efficient GFP targeting into chloroplasts of both dicotyledonous and monocotyledonous plant species with the help of artificial transit peptides (Fig. 4).

### EXAMPLE 2

### Designing and testing transit peptide cleavage site for providing a desired N-terminus (other than alanine) of the protein of interest

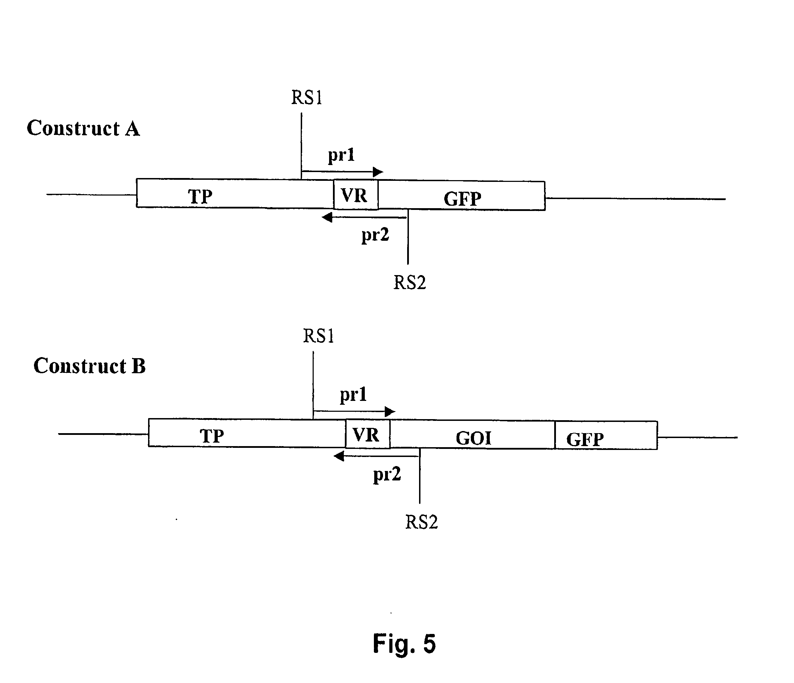

In order to produce a required N-terminus for a protein of interest targeted into the chloroplast, we have analyzed approximately 400 predicted or experimentally identified transit peptide cleavage sites of nuclear-encoded chloroplast targeted proteins from publicly available databases. The results of such analysis are shown in the Table 1. The cleavage sites can be tested for their suitability to provide a desired N-terminus to the protein of interest by using the constructs shown in Figure 5. The simplest version (construct A) consists of a cloning site flanked by a transit peptide (TP) and a reporter gene (GFP). The first methionine (M) of GFP can be replaced by any other amino acid Z in order to find X⁻³X⁻²X⁻¹ triad compatible therewith for generating a cleavage site. Suitable restriction sites RS1 and RS2 can be located within TP and GFP coding regions but not far from each other (preferably within the range of 30-50 bp), thus allowing easy synthesis of two overlapping primers of interest for introducing a desired combination of X⁻³X⁻²X⁻¹ triad and Z amino acid residue in the construct. Prepared constructs can be transiently expressed in the plant cells, GFP compartmentalization can be easily observed under UV-microscopy, and the presence of a required N-terminus can be confirmed by protein microsequencing. In order to facilitate the isolation of GFP, the reporter protein can be tagged at its C-terminal end (e.g. with a 6xHIS-tag). A more elaborated version of the test construct includes the gene of interest-GFP fusion (construt B, Figure 5), as this can provide more precise data for expected results of processing said gene of interest in chloroplasts.

### EXAMPLE 3

### Chloroplast targeting of somatotropin (hGH) and interferon α2B by using artificial transit peptides with cleavage sites according to the invention

The coding sequences for protein fusions shown in the Figure 6 were made and cloned into binary vectors (Fig. 8) according to the standard molecular biology cloning protocols (Sambrook,J., Fritsch, E.F. & Maniatis, T. 1989, Molecular Cloning, A Laboratory Manual, ed 2, CSH Laboratory Press, Cold Spring Harbor, NY). DNA constructs corresponding to the protein fusions shown in Figure 6 are shown in Figure 8. Two fusions with transit peptide for somatotropin and four different fusions for interferon alpha-2b were made in accordance with X⁻³X⁻²X⁻¹ triads of table 1 for the N-terminal amino acids phenylalanine (F) or cysteine (C) for somatotropin and interferon alpha-2b, respectively. The binary vectors shown in Figure 8 are the 3' components (provectors) of tobacco mosaic virus (TMV)-based expression system described in detail in WO 02088369 and by Marillonnet and colleagues (2004, Proc. Natl. Acad. Sci. USA, 101, 6852-6857). *Nicothiana benthamiana* plants were used for the expression of both proteins with the help of provector technology. Alternatively, any appropriate plant expression system can be used in order to achieve the goal of this experiment. In order to check the efficiency of chloroplast targeting and cleaving off of the transit peptide from the targeted protein, the total soluble protein was extracted from leaf material and analysed on Western blots by using commercially available monoclonal antibodies against somatotropin (mouse anti - hGH, Cat.No:RDI-TRK2G2-Gh29, RDI Research Diagnostic, Flanders, NJ, USA) and interferon alpha 2b (Cat. No.95360-0128, Biotrend, Cologne, Germany). The results of Western blot analysis of plastid-targeted hGH using two different X⁻³X⁻²X⁻¹ triads are shown in Fig. 9A. The constructs used for the expression of these two fusions (pICH14061A, X⁻³X⁻²X⁻¹ =R-F-N; pICH14061B, X⁻³X⁻²X⁻¹ =P-S-R) are shown in Fig. 9B.

**Table 1. Combinations of different amino acid sequences providing for transit peptide cleavage sites.**

| *C-terminal end (*X⁻³X⁻²X⁻¹*) of transit peptide* | *N-terminus (Z) of protein of interest* |
|---|---|
| FRV, NRE, VQC, VRC, VPE, SFK, SLT, RFS, RFT | M |
| RGA, SIR, TIV, VRA | C |
| AHS, GST, VHC | I |
| GST, KAT, KQS | N |
| GSD | H |
| VAA | Y |
| PSR, RFN | F |
| IAE, RVA, RSA, SVD | P |
| DDN, IRA, SLG, PGL | Q |
| DSC, IIC, IVC, LRQ, SAT, VHC, VHA, VKC | G |
| IVA, LLV, LPL, LAS, LRQ, MAA, NNN, RTD, TAE, TAQ, TEA, TSE, VAA, VEA, WC | K |
| AAA, IPA, MPT, VPS | R |
| ALA, CRA, IVC, TPS, VRA; | E |
| VLA, LSR | L |
| AGA, CLS, GKR, FPI, IAG, ITC, IVA, KAM, LCM, NMT, PAK, RLR, SVS, TTR, VCM, WA, VAQ, VCC, VRC, VCA, VRA, SLA | S |
| KMS, PRA, PKA, SLF, STS, TGV, TRM, VSF, VRA | V |
| ASA; | D |
| AVA, PAA, VAA, VAG, VSA, VNN, WPR | T |

**Table 2. C-terminal ends (XXX) of transit peptide compatible with more than one N-terminal amino acid residue (Z) of processed protein.**

| *C-terminal end (*X⁻³X⁻²X⁻¹*) of transit peptide* | *N-terminus (*Z*) of protein of interest* |
|---|---|
| VRA | E, V, A, C |
| VAA | T, K, Y |
| VRC | S, M |
| VVA | S, A |
| SLA | S, A |
| GST | N, A |
| AAA | R, A |
| VKC | C,G |
| VHC | G, A |
| VSC | A,C |
| TAQ | *A, K* |

## Claims

1. A process of producing a protein of interest in cells of a predetermined plant, comprising:
(a) selecting the C-terminal three amino acids X⁻³X⁻²X⁻¹ of a transit peptide depending on the N-terminal amino acid Z of said protein of interest according to the correlation table below; and
(b) introducing into said cells a vector encoding a fusion protein comprising from the N-terminus to the C-terminus
(i) said transit peptide for targeting said fusion protein into plastids and, contiguous thereto,
(ii) said protein of interest,
wherein the C-terminal three amino acids X⁻³X⁻²X⁻¹ of said transit peptide and the N-terminal amino acid Z of said protein of interest form a cleavage site
X⁻³X⁻²X⁻¹-Z
for cleaving said fusion protein between X⁻¹ and Z for releasing said protein of interest in plastids;
wherein said correlation table is as follows:
| | |
|---|---|
| **Z** | **X⁻³X⁻²X⁻¹** |
| M | FRV, NRE, VQC, VRC, VPE, SFK, SLT, RFS, or RFT |
| C | RGA, SIR, TIV or VRA |
| I | AHS, GST or VHC |
| F | PSR or RFN |
| P | IAE, RVA, RSA or SVD |
| Q | DDN, IRA, SLG or PGL |
| G | DSC, IIC, IVC, LRQ, SAT, VHC, VHA or VKC |
| K | IVA, LLV, LPL, LAS, LRQ, MAA, NNN, RTD, TAE, TAQ, TEA, TSE, VAA, VEA, or VVC |
| R | AAA, IPA, MPT or VPS |
| Y | VAA |
| H | GSD |
| N | GST, KAT or KQS |
| S | AGA, CLS, GKR, FPI, IAG, ITC, IVA, KAM, LCM, NMT, PAK, RLR, SVS, SLA, TTR, VCM, WA, VAQ, VCC, VRC, VCA, or VRA |
| V | KMS, PRA, PKA, SLF, STS, TGV, TRM, VSF or VRA |
| D | ASA |
| E | ALA, CRA, IVC, TPS or VRA |
| L | VLA or LSR |
| T | AVA, PAA, VAA, VAG, VSA, VNN or WPR |
wherein all triads of amino acids are given in N-terminal to C-terminal direction and amino acids are given in the one-letter amino acid code.

2. The process according to claim 1, wherein the amino acid sequence X⁻³X⁻²X⁻¹ of the correlation table of claim 1 is selected from the set of amino acid sequences X⁻³X⁻²X⁻¹ naturally occurring contiguous to Z in plastid-targeted fusion proteins in the family of plants said predetermined plant is a member of.

3. The process according to claim 1, wherein the amino acid sequence X⁻³X⁻²X⁻¹ of the correlation table of claim 1 is selected from the set of amino acid sequences X⁻³X⁻²X⁻¹ naturally occurring contiguous to Z in plastid-targeted fusion proteins in the genus of plants said predetermined plant is a member of.

4. The process according to claim 1, wherein the amino acid sequence X⁻³X⁻²X⁻¹ of the correlation table of claim 1 is selected from the set of amino acid sequences X⁻³X⁻²X⁻¹ naturally occurring contiguous to Z in plastid-targeted
fusion proteins of plants of the same species as said predetermined plant.

5. The process according to any one of claims 1 to 4, wherein the transit peptide has the sequence MASSMLSSAA VVATRASAAQ ASMVAPFTGL KSAASFPVTR KQNNLDITSI ASNGGR upstream of the cleavage site.

6. The process according to any one of claims 1 to 4, wherein the transit peptide has the sequence MAPTVMASSA TTVAPFQGLK STAGRLPVAR RSSGSLGSVS NGGR upstream of the cleavage site.

7. The process according to any one of claims 1 to 6, wherein said protein of interest is of bacterial, viral, plant, or animal origin.

8. The process according to any one of claims 1 to 6, wherein said protein of interest is a polypeptide hormone.

9. The process according to any one of claims 1 to 8, wherein said protein of interest is an interferon.

## Patentansprüche

1. Verfahren zur Herstellung eines gewünschten Proteins in Zellen einer vorbestimmten Pflanze, umfassend:
(a) Auswählen der C-terminalen drei Aminosäuren X⁻³X⁻²X⁻¹ eines Transitpeptids abhängig von der N-terminalen Aminosäure Z des gewünschten Proteins gemäß der unten stehenden Korrelationstabelle und
(b) Einführen eines Vektors in die Zellen, wobei der Vektor für ein Fusionsprotein kodiert, das vom N-Terminus zum C-Terminus aufweist:
(i) das Transitpeptid zum Leiten (targeting) des Fusionsprotein in Plastiden und angrenzend dazu
(ii) das gewünschte Protein,
wobei die C-terminalen drei Aminosäuren X⁻³X⁻²X⁻¹ des Transitpeptids und die N-terminale Aminosäure Z des gewünschten Proteins eine Spaltstelle
X⁻³X⁻²X⁻¹-Z
bilden, zum Spalten des Fusionsproteins zwischen X⁻¹ und Z und zum Freisetzen des gewünschten Proteins in Plastiden,
wobei die Korrelationstabelle wie folg lautet:
| | |
|---|---|
| **Z** | **X⁻³X⁻²X⁻¹** |
| M | FRV, NRE, VQC, VRC, VPE, SFK, SLT, RFS oder RFT |
| C | RGA, SIR, TIV oder VRA |
| I | AHS, GST oder VHC |
| F | PSR oder RFN |
| P | IAE, RVA, RSA oder SVD |
| Q | DDN, IRA, SLG oder PGL |
| G | DSC, IIC, IVC, LRQ, SAT, VHC, VHA oder VKC |
| K | IVA, LLV, LPL, LAS, LRQ, MAA, NNN, RTD, TAE, TAQ, TEA, TSE, VAA, VEA oder VVC |
| R | AAA, IPA, MPT oder VPS |
| Y | VAA |
| H | GSD |
| N | GST, KAT oder KQS |
| S | AGA, CLS, GKR, FPI, IAG, ITC, IVA, KAM, LCM, NMT, PAK, RLR, SVS, SLA, TTR, VCM, WA, VAQ, VCC, VRC, VCA oder VRA |
| V | KMS, PRA, PKA, SLF, STS, TGV, TRM, VSF oder VRA |
| D | ASA |
| E | ALA, CRA, IVC, TPS oder VRA |
| L | VLA oder LSR |
| T | AVA, PAA, VAA, VAG, VSA, VNN oder WPR |
worin alle Aminosäuretriaden in Richtung vom N-Terminus zum C-Terminus angegeben sind und Aminosäuren im Ein-Buchstaben-Aminosäure-Code angeben sind.

2. Das Verfahren gemäß Anspruch 1, wobei die Aminosäuresequenz X⁻³X⁻²X⁻¹ der Korrelationstabelle von Anspruch 1 ausgewählt ist aus der Menge von Aminosäuresequenzen X⁻³X⁻²X⁻¹ , die natürlicherweise angrenzend an Z in plastidengeleiteten (plastid-targeted) Fusionsproteinen in der Pflanzenfamilie, zu der die vorbestimmte Pflanze gehört, vorkommen.

3. Das Verfahren gemäß Anspruch 1, wobei die Aminosäuresequenz X-³X-²X-¹ der Korrelationstabelle von Anspruch 1 ausgewählt ist aus der Menge von Aminosäuresequenzen X⁻³X⁻²X⁻¹, die natürlicherweise angrenzend an Z in plastidengeleiteten (plastid-targeted) Fusionsproteinen in der Pflanzengattung, zu der die vorbestimmte Pflanze gehört, vorkommen.

4. Das Verfahren gemäß Anspruch 1, wobei die Aminosäuresequenz X⁻³X⁻²X⁻¹ der Korrelationstabelle von Anspruch 1 ausgewählt ist aus der Menge von Aminosäuresequenzen X⁻³X⁻²X⁻¹ , die natürlicherweise angrenzend an Z in plastidengeleiteten (plastid-targeted) Fusionsproteinen in der Pflanzenart, zu der die vorbestimmte Pflanze gehört, vorkommen.

5. Das Verfahren gemäß einem der Ansprüche 1 bis 4, wobei das Transitpeptid die Sequenz MASSMLSSAA VVATRASAAQ ASMVAPFTGL KSAASFPVTR KQNNLDITSI ASNGGR stromaufwärts der Spaltstelle aufweist.

6. Das Verfahren gemäß einem der Ansprüche 1 bis 4, wobei das Transitpeptid die Sequenz MAPTVMASSA TTVAPFQGLK STAGRLPVAR RSSGSLGSVS NGGR stromaufwärts der Spaltstelle aufweist.

7. Das Verfahren gemäß einem der Ansprüche 1 bis 6, wobei das gewünschte Protein bakteriellen, viralen, pflanzlichen oder tierischen Ursprungs ist.

8. Das Verfahren gemäß einem der Ansprüche 1 bis 6, wobei das gewünschte Protein ein Polypeptidhormon ist.

9. Das Verfahren gemäß einem der Ansprüche 1 bis 8, wobei das gewünschte Protein ein Interferon ist.

## Revendications

1. Procédé de production d'une protéine présentant un intérêt dans des cellules d'une plante prédéterminée, comprenant :
(a) la sélection des trois acides aminés C-terminaux X⁻³X⁻²X⁻¹ d'un peptide de transit en fonction de l'acide aminé N-terminal Z de ladite protéine présentant un intérêt selon le tableau de corrélation ci-dessous ; et
(b) l'introduction dans lesdites cellules d'un vecteur codant une protéine de fusion comprenant, de l'extrémité N à l'extrémité C
(i) ledit peptide de transit pour cibler ladite protéine de fusion dans des plastides et, contigu à celui-ci,
(ii) ladite protéine présentant un intérêt,
dans lequel les trois acides aminés C-terminaux X⁻³X⁻²X⁻¹ dudit peptide de transit et l'acide aminé N-terminal Z de ladite protéine présentant un intérêt forment un site de coupure
X⁻³X⁻²X⁻¹-Z
pour une coupure de ladite protéine de fusion entre X⁻¹ et Z afin de libérer ladite protéine présentant un intérêt dans les plastides ;
dans lequel ladite table de corrélation est la suivante :
| | |
|---|---|
| Z | X⁻³X⁻²X⁻¹ |
| M | FRV, NRE, VQC, VRC, VPE, SFK, SLT, RFS, ou RFT |
| C | RGA, SIR, TIV ou VRA |
| I | AHS, GST ou VHC |
| F | PSR ou RFN |
| P | IAE, RVA, RSA ou SVD |
| Q | DDN, IRA, SLG ou PGL |
| G | DSC, IIC, IVC, LRQ, SAT, VHC, VHA ou VKC |
| K | IVA, LLV, LPL, LAS, LRQ, MAA, NNN, RTD, TAE, TAQ, TEA, TSE, VAA, VEA ou VVC |
| R | AAA, IPA, MPT ou VPS |
| Y | VAA |
| H | GSD |
| N | GST, KAT ou KQS |
| S | AGA, CLS, GKR, FPI, IAG, ITC, IVA, KAM, LCM, NMT, PAK, RLR, SVS, SLA, TTR, VCM, VVA, VAQ, VCC, VRC, VCA, ou VRA |
| V | KMS, PRA, PKA, SLF, STS, TGV, TRM, VSF ou VRA |
| D | ASA |
| E | ALA, CRA, IVC, TPS ou VRA |
| L | VLA ou LSR |
| T | AVA, PAA, VAA, VAG, VSA, VNN ou WPR |
où toutes les triades d'acides aminés sont indiquées dans la direction de l'extrémité N à l'extrémité C, et les acides aminés sont indiqués selon le code d'acide aminé à une seule lettre.

2. Procédé selon la revendication 1, dans lequel la séquence d'acides aminés X⁻³X⁻²X⁻¹ de la table de corrélation de la revendication 1 est choisie dans l'ensemble de séquences d'acides aminés X⁻³X⁻²X⁻¹ naturels contigus à Z dans des protéines de fusion ciblées par un plastide dans la famille de plantes dont ladite plante prédéterminée est un membre.

3. Procédé selon la revendication 1, dans lequel la séquence d'acides aminés X⁻³X⁻²X⁻¹ de la table de corrélation de la revendication 1 est choisie dans l'ensemble de séquences d'acides aminés X⁻³X⁻²X⁻¹ naturels contigus à Z dans des protéines de fusion ciblées par un plastide dans le genre de plantes dont ladite plante prédéterminée est un membre.

4. Procédé selon la revendication 1, dans lequel la séquence d'acides aminés X⁻³X⁻²X⁻¹ de la table de corrélation de la revendication 1 est choisie dans l'ensemble de séquences d'acides aminés X⁻³X⁻²X⁻¹ naturels contigus à Z dans des protéines de fusion ciblées par un plastide de plantes de la même espèce que ladite plante prédéterminée.

5. Procédé selon l'une quelconque des revendications 1 à 4, dans lequel le peptide de transit a la séquence MASSMLSSAA WATRASAAQ ASMVAPFTGL KSAASFPVTR KQNNLDITSI ASNGGR en amont du site de coupure.

6. Procédé selon l'une quelconque des revendications 1 à 4, dans lequel le peptide de transit a la séquence MAPTVMASSA TTVAPFQGLK STAGRLPVAR RSSGSLGSVS NGGR en amont du site de coupure.

7. Procédé selon l'une quelconque des revendications 1 à 6, dans lequel ladite protéine présentant un intérêt est d'origine bactérienne, virale, végétale ou animale.

8. Procédé selon l'une quelconque des revendications 1 à 6, dans lequel ladite protéine présentant un intérêt est une hormone polypeptidique.

9. Procédé selon l'une quelconque des revendications 1 à 8, dans lequel ladite protéine présentant un intérêt est un interféron.
